# EUROPÄISCHE PATENTSCHRIFT

(11) **EP 0 521 022 B1**
(45) Veröffentlichungstag und Bekanntmachung des Hinweises auf die Patenterteilung: **21.08.1996**
(21) Anmeldenummer: 91905792.7
(22) Anmeldetag: 12.03.1991
(51) Int. Cl.: B05B 11/02, B05B 11/00, A61M 5/315, A61M 5/24

(54) **AUSTRAGVORRICHTUNG FÜR MEDIEN**
APPLICATOR FOR MEDIA
APPLICATEUR POUR MILIEUX

(30) Priorität: 14.03.1990 DE 4008068
(43) Veröffentlichungstag der Anmeldung: 07.01.1993
(73) Patentinhaber: Ing. Erich Pfeiffer GmbH, 78315 Radolfzell (DE)
(72) Erfinder: FUCHS, Karl-Heinz, D-7760 Radolfzell (DE)
(74) Vertreter: Patentanwälte Ruff, Beier, Schöndorf und Mütschele
(86) Internationale Anmeldenummer: EP9100457
(87) Internationale Veröffentlichungsnummer: WO9113689

(56) Entgegenhaltungen:
- EP-A- 0 334 349
- FR-A- 2 242 113
- FR-A- 2 300 916

## Beschreibung

Die Erfindung betrifft eine Austragvorrichtung für insbesondere fließfähige Medien, die gasförmig bzw. leicht flüchtig, flüssig bzw. pastös, pulverförmig und/oder ähnlich sein können. Sie kann erfindungsgemäß mindestens eine handbetätigbare Pumpe und eine mit deren Pumpenkammer über einen Auslaßkanal verbundene Austragöffnung, wie eine Zerstäuberdüse aufweisen, so daß durch Handbetätigung ein einfacher Austrag mindestens eines vorbestimmten Medienquantums gewahrleistet ist.

Aus der EP-A-0 334 349 ist eine Einweg-Sprühvorrichtung bekanntgeworden, bei der eine Injektionsspritze in eine Sprühvorrichtung eingesetzt wird, die aus zwei ineinanderschiebbaren Gehäusehälften besteht. Die untere, äußere Gehäusehälfte weist einen mit Einschnürungen oder Stufen versehenen Schlitz auf, in dem ein Vorsprung der anderen Gehäusehälfte läuft und bei Weg der Spitze in mehrere Teilhübe aufzuteilen gestattet.

Der Erfindung liegt die Aufgabe zugrunde, Nachteile bekannter Lösungen zu vermeiden und insbesondere eine handbetätigbare Austragvorrichtung für die aufeinanderfolgende Ausgabe mehrerer Medienquanten zu schaffen, die für Verschmutzung bzw. Kontaminierung wenig zugänglich ist. Diese Aufgabe wird durch die kennzeichnenden Merkmale von Anspruch 1 gelöst.

Es sind also Mittel vorgesehen, um mindestens einen Teilhub an seinem Hubende so zu begrenzen, daß auf einen weiterführenden Teilhub umgeschaltet werden kann, wobei vorzugsweise die nach außen dicht geschlossene und einlaßfreie Pumpenkammer die gesamte, mit ihr auszutragende Medienmenge speichert und durch Verengung in den Teilhüben ohne Nachfüllung entleert wird. Die Ausbildung eignet sich daher insbesondere für Austragvorrichtungen nach der deutschen Patentanmeldung P 35 32 890.8, auf die wegen weiterer Einzelheiten und Wirkungen Bezug genommen wird.

Zur Begrenzung des jeweiligen Teilhubes ist eine Arretiervorrichtung mit Anschlägen der gegeneinander bewegbaren, insbesondere verschiebbaren Bauteile vorgesehen. Auch kann der eine Bauteil bzw. die an diesem vorgesehene Handhabe am Ende eines Teilhubes und vor Erreichen der Pumphub-Endstellung eine solche Lage gegenüber dem anderen Bauteil einnehmen, daß er ohne Umgreifen nicht oder nur sehr schwer weiter betätigt werden könnte. Ist z.B. die Handhabe des zuerst genannten Bauteiles durch den Quersteg eines Bügels bzw. die Stirnwand einer Kappe gebildet, so kann diese durch einen etwa radial zu ihr liegenden Daumen soweit betätigt werden, bis sie etwa bündig mit dem zugehörigen Gehäuseende des anderen Bauteiles abschließt. Für eine weitere Betätigung bzw. einen weiteren Teilhub, bei welchem die Handhabe des einen Bauteiles in den anderen vollständig versenkt wird, muß mit dem Daumen dann so umgegriffen werden, daß er etwa axial liegt.

Die Arretiervorrichtung ist zwischen zwei Gehäuseteilen vorgesehen, von denen wenigstens das versenkbare im wesentlichen formstabil ist und die vorzugsweise ein vollständig geschlossenes Außengehäuse für die gesamte Austragvorrichtung bzw. die Pumpe bilden, über welches ggf. nur noch ein stutzenförmiger oder ähnlicher Austragfortsatz vorsteht, der die Austragöffnung aufweist. Der größere der beiden Gehäuseteile ist zweckmäßig mit dem Austragstutzen verbunden bzw. im wesentlichen einteilig ausgebildet.

Die Arretierung kann raumsparend und leicht montierbar im Bereich des hinteren Endes der Austragvorrichtung, z.B. in Ausgangsstellung hinter dem der Austragöffnung zugekehrten Ende der Pumpkammer, vorgesehen sein. Ferner kann für die Überführung von einem Teilhub zum nächsten eine Um- bzw. Folgeschaltung z.B. nach Art der Umschaltung eines nur mit einem einzigen Druckknopf betätigbaren Mehrfarben-Schreibstiftes vorgesehen sein, so daß die Pumpe zwischen zwei Teilhüben nicht in die Ausgangsstellung zurückgeführt werden muß. Wird sie in diese Ausgangsstellung zurückgeführt, so kann dies in vorteilhafter Weise zum Leersaugen des Auslaßkanales und ggf. zum Ansaugen von Luft in die Pumpenkammer herangezogen werden. Es ist aber auch denkbar, trotz Rückführung der Bauteile bzw. Handhaben in die Ausgangsstellung durch Vorsehen einer Art Freilaufes hierbei eine Rückführung der Pumpe in die Ausgangsstellung zu verhindern.

Als Pumpe ist bevorzugt eine Schubkolbenpumpe mit einem einfachen, formstabilen Speicher- bzw. Zylinderbehälter vorgesehen, dessen Mantel mit seinem Boden einteilig ausgebildet sein kann. Ist dieser Zylinderbehälter in eine Schiebeführung des einen Bauteiles anschlagbegrenzt eingesetzt, deren Reibung gegenüber dem Behälter kleiner als diejenige des Pumpkolbens oder eines mit diesem verbundenen Bauteiles ist, so bleibt der Pumpzylinder am Ende jedes Teilhubes gegenüber dem Pumpkolben im wesentlichen stehen, während der die Schiebeführung aufweisende Bauteil mit der zugehörigen Handhabe in Richtung zur Ausgangsstellung zurückgeführt werden kann.

Die vollständig und mit Radialabstand innerhalb des Außengehäuses liegende Pumpe, deren Pumpkolben und Zylinder in jeder Stellung zwischen den Stirnwänden dieses Außengehäuses liegen können, kann zusätzlich geschützt innerhalb eines Innenkäfigs bzw. Innengehäuses vorgesehen sein, das mit Radialabstand innerhalb des Außengehäuses liegt und sich ggf. im wesentlichen über dessen gesamte Länge erstreckt. Dieses Innengehäuse kann, wie das Außengehäuse, aus zwei mit ihren offenen Seiten teleskopartig ineinandergreifenden Kappen bestehen, die in Ausgangsstellung anschlagbegrenzt ineinandergreifen, ggf. eine Führung für eine innerhalb eines Ringraumes liegende Rückstellfeder bilden und/oder durch eine Schnappverbindung oder dgl. miteinander zu verbinden sind, welche die einzige, gegen versehentliches Lösen wirkende Axialsicherung der beiden Bauteile gegeneinander nach der Montage bildet.

Diese und weitere Merkmale von bevorzugten Weiterbildungen der Erfindung gehen außer aus den Ansprüchen auch aus der Beschreibung und den Zeichnungen hervor, wobei die einzelnen Merkmale jeweils für sich allein oder zu mehreren in Form von Unterkombinationen bei einer Ausführungsform der Erfindung und auf anderen Gebieten verwirklicht sein und vorteilhafte sowie für sich schutzfähige Ausführungen darstellen können. Ausführungsbeispiele der Erfindung sind in den Zeichnungen dargestellt und werden im folgenden näher erläutert. In den Zeichnungen zeigen:

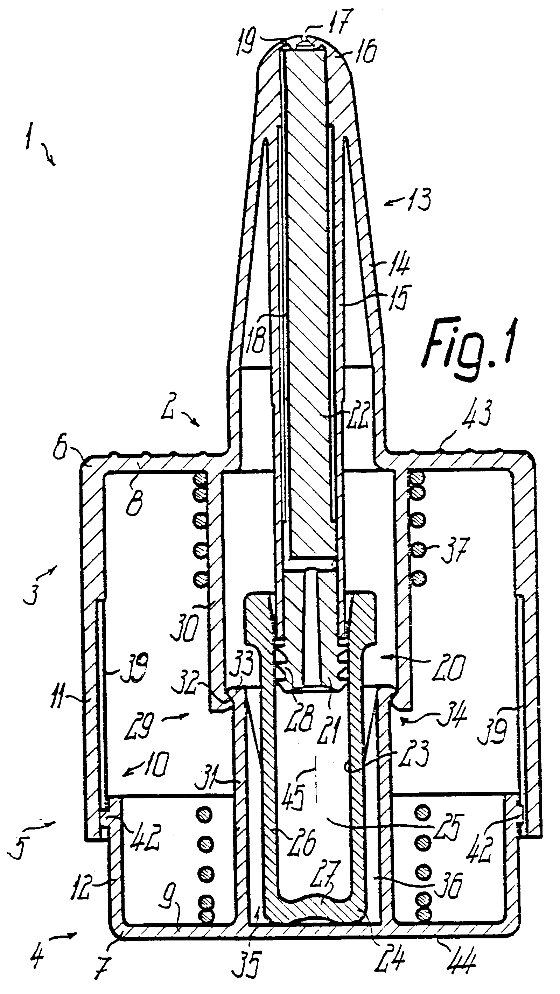

- Fig. 1: eine erfindungsgemäße Austragvorrichtung im Axialschnitt,
- Fig. 2: einen Ausschnitt der Fig. 1 in Ansicht,
- Fig. 3: den Ausschnitt gemäß Fig. 2 im Querschnitt und
- Fig. 4: eine weitere Ausführungsform in einer Darstellung entsprechend Fig. 2.

Die Austragvorrichtung 1 weist einen im wesentlichen einteiligen Grundkörper 2 auf, der einen von zwei gegeneinander axial verschiebbaren Bauteilen 3, 4 bildet. Gemeinsam bilden beide Bauteile ein im wesentlichen geringfügig abgestuft zylindrisches Außengehäuse 5, dessen Länge in der Größenordnung seines Durchmessers von z.B. nur etwa 30 mm liegt. Das dünnwandige Gehäuse 3 wird von zwei kappenförmigen Gehäuseteilen 6, 7 begrenzt, von denen der des Grundkörpers 2 den anderen Gehäuseteil 7 am Außenumfang eng umgibt. Jeder Gehäuseteil 6 bzw. 7 weist eine annähernd ebene Stirnwand 8 bzw. 9 und einen annähernd zylindrischen Mantel 11 bzw. 12 auf. Den Mänteln 11, 12 ist eine Arretierung 10 zugeordnet, welche den Hub der beiden Gehäuseteile 6, 7 gegeneinander je nach Drehstellung unterschiedlich unmittelbar begrenzt.

An der Außenseite der Stirnwand 8 des Gehäuseteiles 6 steht im Zentrum ein im Durchmesser wesentlich reduzierter Stutzen 13 vor, der im wesentlichen durch eine mit einem Ende einteilig in die Stirnwand 8 übergehende Außenhülse 14 und ein innerhalb dieser im wesentlichen berührungsfrei liegendes Innenrohr 15 gebildet ist, die an ihren vorderen Enden zur Bildung einer Düsenkappe 16 einteilig ineinander übergehen. In der Stirnwand der Düsenkappe 16 ist eine ins Freie führende Austragöffnung 17 vorgesehen, die unter Zwischenschaltung einer Dralleinrichtung 19 an ein Ende eines Auslaßkanales 18 angeschlossen ist.

Innerhalb des Außengehäuses 5 ist eine Pumpe 20 aus einteiligem Pumpkolben 21 mit Kolbenschaft 22 und Zylinder 23 angeordnet, der durch einen nahezu vollständig geschlossenen, mit dem Medium gefüllten Behälter 24 gebildet ist. Außer den Speicherraum bildet das Innere auch die an einem Ende ausschließlich vom Pumpkolben 21 dicht verschlossene Pumpenkammer 25. Der Behälter 24 ist durch einen im wesentlichen zylindrischen Mantel 26 und einen Boden 27 gebildet, dessen Kolbenlaufbahn am offenen, mit einem vorstehenden Außenrand versehenen Ende auf voller Weite offen bzw. trichterförmig erweitert ist, ohne daß ein Zylinderdeckel oder dgl. vorgesehen wäre.

Der Kolbenschaft 22 bildet mit seinem vom Pumpkolben 21 abgekehrten Ende einen Bestandteil der Dralleinrichtung 19 und eine Stirnbegrenzung des inneren Endes der Auslaßöffnung 17. Der Auslaßkanal 18 ist im Anschluß an die Pumpenkammer 25 innerhalb des Pumpkolbens 21 vorgesehen und geht dann über einen Querkanal in einen Abschnitt über, der zwischen dem Außenumfang des Kolbenschaftes 22 und dem Innenumfang der Innenhülse 15 liegt. Der Kolbenschaft 22 weist zwei abgestuft zur Auslaßöffnung 17 im Außendurchmesser abnehmende Schaftabschnitte auf, von denen der an den Pumpkolben 21 anschließende eng in der Bohrung der Innenhülse 15 liegt, die im Abstand von diesem Abschnitt nahe beim zugehörigen Schaftende ebenfalls entsprechend abgestuft ist, so daß sie dieses Schaftende eng umschließt, dazwischen aber einen geringfügigen Spaltabstand vom Außenumfang des Kolbenschaftes 22 hat. Der Pumpkolben 21 weist mehrere, z.B. drei unmittelbar benachbart hintereinander liegende annähernd spitzwinklig bzw. sägezahnförmig flankierte, nur durch entsprechend spitzwinklige Ringnuten voneinander getrennte und einteilig mit ihm ausgebildete Kolbenlippen 28 auf, deren schräge Flanken jeweils ihre vorderen Flanken bilden und die mit Vorspannung am Innenumfang des Zylinders 23 anliegen. Die Innenhülse 15, die die Stirnwand 8 berührungsfrei durchsetzt und in das Außengehäuse 5 frei hineinragt, kann bis in den Zylinder 23 bis nahe an das hintere Ende des Pumpkolbens 21 hineinreichen und am Innenumfang des Zylinders 23 gleiten.

In dem Außengehäuse 5 ist mit Umfangsabstand ein kleineres, jedoch etwa gleichlanges, zylindrisch abgestuftes Innengehäuse 29 angeordnet, das im wesentlichen aus einer Außenhülse 30 und einer Innenhülse 31 besteht. Die Außenhülse 30, die nur geringfügig weiter als die Außenhülse 14 ist, steht einteilig über die Innenseite der Stirnwand 8 weniger weit als der Mantel 11 vor und ist durchgehend zum Innenraum der Außenhülse 14 offen. Die Innenhülse 31 schließt mit ihrem Mantel entsprechend unmittelbar an die Innenseite der Stirnwand 9 einteilig an und steht axial weiter als der Mantel 12 und daher über dessen Endfläche vor. An ihren freien Enden weisen die Hülsen jeweils einen ringbundförmigen Anschlag 32 bzw. 33 für den gegenseitigen Eingriff so auf, daß der Bauteil 4 in der Pumphub-Ausgangsstellung axial nicht vom anderen Bauteil 3 abgezogen werden kann. Gleichzeitig bilden diese Anschläge eine selbstaufweitende Schnappverbindung 34 zum federrastartigen Verbinden der beiden Bauteile 3, 4 bei der Montage. Das Ende des Pumpkolbens 21 ist gegenüber der Endfläche der Außenhülse 30 geringfügig zurückversetzt.

Die Innenhülse 31 bildet eine Schiebe- bzw. Steck-Aufnahme 35 für den über die inneren Endflächen des Mantels 12 und der Innenhülse 31 vorstehenden Behälter 24, der in Montagelage am Boden der Innenhülse 31 bzw. an der Innenseite der Stirnwand 9 abgestützt anliegt und der ansonsten nur noch am Außenumfang gesichert ist. Zu diesem Zweck liegt der Behälter 24 nicht am Innenumfang der Innenhülse 31, sondern an gleichmäßig über den Umfang verteilten Radial-Rippen 36 bzw. an deren radial inneren Kantenflächen an. Ist diese Anlage mit Vorspannung vorgesehen, so führt der Behälter 24 alle Bewegungen gemeinsam mit dem Bauteil 4 aus; die Anlage kann aber auch eine Schiebeführung bilden, so daß der Behälter 24 nach Art einer Schleppkoppelung bei Rückhubbewegungen des Bauteiles 4 stets diejenige Lage beibehält, die er gegenüber dem Pumpkolben 21 erreicht hat.

Es ist auch denkbar, den Behälter 24 im wesentlichen freistehend einteilig mit dem Bauteil 4 auszubilden und auf das Innengehäuse 29 zu verzichten. In der Pumphub-Endstellung liegt das offene, über die Innenhülse 31 vorstehende Ende des Behälters 24 nahezu vollständig innerhalb der Außenhülse 14, in der es dann als Rückzugsicherung mit seinem Außenrand festgeklemmt sein kann. Die Endfläche der Innenhülse 31 liegt mit geringem Spaltabstand von der Innenfläche der Stirnwand 8 und die Bodenwand 9 mit entsprechendem Spaltabstand von der Endfläche der Außenhülse 30. Dadurch ist sichergestellt, daß die innere Stirnfläche des Pumpkolbens 21 bis an den Boden 27 des Behälters 24 heranbewegt werden kann und die Pumpbewegung nur dadurch anschlagbegrenzt ist.

Die Arretierung 10 weist mehrere, z.B. zwei gleichmäßig über den Umfang verteilte Nocken 42 auf, die nahe beim offenen Ende des Gehäuseteiles 7 über dessen Außenumfang vorstehen. Jedem Nocken 42 ist eine der Anzahl von Teilhüben entsprechende Anzahl von unmittelbar benachbart zueinander liegenden Längsführungen 38, 39 zugeordnet, die durch Nuten im Innenumfang des Mantels 11 gebildet sind, welche von dessen offener Endfläche ausgehen und nur über einen Teil seiner Länge reichen. Die Endfläche 41 der kürzeren Längsführung 38 bildet einen Anschlag für den Nocken 42 am Ende des ersten Teilhubes, nach welchem etwa die Hälfte der Speichermenge von etwa 0,1 ml aus dem Behälter 24 ausgetragen ist. Am Ende dieses Teilhubes liegt die Außenseite der Stirnwand 9 des Gehäuseteiles 7 etwa in einer Ebene mit der zugehörigen Endfläche des Gehäuseteiles 6. Benachbart zum bei dieser Endfläche liegenden Anfangsende sind die Längsführungen 38, 39 über eine Querführung 40 miteinander verbunden.

Am Ende des ersten Teilhubes wird der Bauteil 4 durch eine im Ringraum zwischen den beiden Gehäusen liegende und sich an den Stirnwänden 8, 9 abstützende Rückstellfeder 37 mit dem Nocken 42 zur Ausgangsstellung zurückgeführt, so daß durch Verdrehen des Bauteiles 4 gegenüber dem Bauteil 3 der Nocken 42 über die Querführung 40 an das Anfangsende der Längsführung 39 gebracht werden kann. Hierbei schlägt der Nocken 42 an der zugehörigen Flanke der Längsführung 39 an, da die entsprechenden Flanken der Längsführungen 38, 39 beiderseits an den Enden der Querführung 40 liegen. Nun kann in einem erneuten Hub die Restmenge aus dem Behälter 24 gepumpt werden, wobei jedoch der Nocken 42 zweckmäßig nicht an der Endfläche der Längsführung 39 anschlägt. Aus dieser Endstellung kann der Gehäuseteil 7 wieder in seine Ausgangsstellung zurückkehren.

Die Außenseite der Stirnwand 8 bildet eine ringförmig den Stutzen 13 umgebende Druck-Handhabe 43, während die Außenseite der Stirnwand 9 eine Druck-Handhabe 44 bildet, deren Außendurchmesser nahezu gleichgroß wie derjenige der Handhabe 43 ist. Die Austragvorrichtung 1 wird in der Regel mit drei Fingern betätigt, wobei Zeige- und Mittelfinger an der Handhabe 43 anliegen. Im wesentlichen alle Bauteile und Ausbildungen liegen in einer gemeinsamen Mittelachse 45 bzw. symmetrisch zu dieser.

Auch in der Ausgangsstellung kann der Bauteil 4 gegen Pumpbewegungen auslösbar arretiert sein. Z.B. kann die zugehörige, zwischen den Längsführungen 38, 39 liegende Querbegrenzung der Querführung 40 eine Anschlagfläche 46 für den Nocken 42 bilden, so daß vor Ingebrauchnahme der Nocken 42 verdreht werden muß. Auch die Nocken 42 könnten zur Herstellung der Schnappverbindung zwischen den beiden Bauteilen 3, 4 bzw. zu deren axialer Lösesicherung in der Ausgangsstellung vorgesehen sein. In diesem Fall wären die Längsführungen 38, 39 sowie ggf. die Querführung 40 an ihren zugehörigen, bei der Endfläche des Mantels 11 liegenden Enden geschlossen.

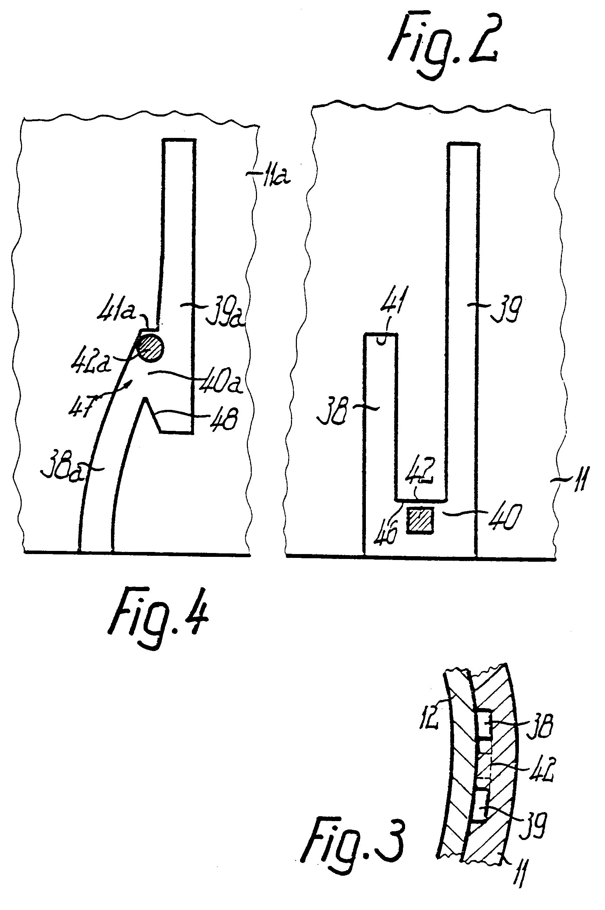

Auf eine Rückstellfeder 37 könnte auch vollständig verzichtet werden. Z.B. kann gemäß Fig. 4 die Querführung 40a im Anschluß an die Endfläche 41a der Längsfläche 38a vorgesehen sein, so daß der Nocken 42a ohne eine axiale Rückstellbewegung direkt in die folgende Längsführung 39a gedreht werden kann. Insbesondere wenn in diesem Fall eine Rückstellfeder 37 vorgesehen ist, kann durch eine kleine, axiale Rückstellbewegung erreicht werden, daß der Nocken 42a unter der Kraft der Rückstellfeder auf eine geneigte Steuerflanke 48 aufläuft und dadurch ausgerichtet von selbst in den Anfang der Längsführung 39a überführt wird. Dadurch ergibt sich eine Folgeschaltung 47.

Durch die jeweilige Lage der Bauteile 3, 4 gegeneinander kann erkannt werden, wieviel Teilhübe ausgeführt worden sind, so daß sich auf einfache Weise eine Anzeigevorrichtung ergibt.

Der Bauteil 4 bildet mit dem Behälter 24 eine Montageeinheit, die in einem einzigen Steckvorgang mit der anderen Montageeinheit verbunden werden kann, die aus dem Bauteil 3 und dem Pumpkolben 21 besteht. Dadurch kann die Austragvorrichtung 1 sehr gut gefüllt und zusammengesetzt bzw. geschlossen werden.

## Patentansprüche

1. Austragvorrichtung für Medien mit einer handbetätigbaren Pumpe (20), die je gesamtem, zwischen einer Ausgangsstellung und einer Pumphub-Endstellung liegendem Pumphub in Teilhüben zweier gegeneinander bewegbarer Bauteile (3, 4) eines Gehäuses (5) betätigbar ist und am Ende mindestens eines Teilhubes eine den Pumphub festsetzende Arretierung (10) vorgesehen ist, die unmittelbar im Anschluß an den Teilhub lösbar und aus zusammenwirkenden Vorsprüngen (42) und Ausnehmungen (39) der Bauteile (3, 4) des Gehäuses (5) gebildet ist, wobei die Vorsprünge und Ausnehmungen verdeckt im Inneren des von dem Gehäuse (5) abgegrenzten Raumes angeordnet sind.

2. Austragvorrichtung nach Anspruch 1, dadurch gekennzeichnet, daß die Arretierung (10) für zwei Druck-Handhaben (43, 44) für die Pumpenbetätigung vorgesehen ist, deren Druckflächen quer zu einer Pumpenachse (45) und/oder an unmittelbar ineinandergreifenden Bauteilen (3, 4) vorgesehen sind, daß vorzugsweise die Arretierung (10) zwischen zwei kappenförmigen Gehäuseteilen (6, 7) vorgesehen ist, die insbesondere mit entgegengesetzt gerichteten Kappenmänteln (11, 12) ineinandergreifen und/oder mit Kappen-Stirnwänden (8, 9) Handhaben (43, 44) bilden und daß vorzugsweise die Arretierung (10) einem Gehäuseteil (7) eines Außengehäuses (5) zugeordnet ist, der einen hinteren Endteil der Austragvorrichtung (1) bildet, teleskopartig in einen vorderen Gehäuseteil (6) eingreift und/oder eine hintere, im wesentlichen über den Gesamtquerschnitt der Austragvorrichtung reichende Endfläche bildet.

3. Austragvorrichtung nach Anspruch 1 oder 2, dadurch gekennzeichnet, daß die Arretierung (10) im Bereich eines hinteren Endes der Austragvorrichtung (1) und/oder hinter einem vorderen Ende einer Pumpenkammer (25) vorgesehen ist, zu der die Arretierung (10) insbesondere radial benachbart liegt.

4. Austragvorrichtung nach einem der vorhergehenden Ansprüche, dadurch gekennzeichnet, daß vorzugsweise der in Ausgangsstellung ein hinteres Ende der Austragvorrichtung (1) bildende Bauteil (4) über den gegenüber ihm arretierbaren Bauteil (3) übersteht, in Pumphub-Endstellung zumindest am Ende eines Teilhubes im wesentlichen vollständig innerhalb dieses Bauteiles (3) liegt, wobei die Bauteile (3, 4) in dieser Stellung insbesondere annähernd miteinander fluchtende Endflächen bilden.

5. Austragvorrichtung nach einem der vorhergehenden Ansprüche, dadurch gekennzeichnet, daß die Pumpe (20) als Schubkolbenpumpe, mit einem in einem Pumpenzylinder (23) verschiebbar eingreifenden Pumpkolben (21) ausgebildet ist und/oder daß der Pumpenzylinder (23) ein am Boden (27) geschlossener Speicher-Behälter (24) für den gesamten Medien-Speicherinhalt für die Pumpe (20) ist, der lediglich mit dem Pumpkolben (21) geschlossen ist und durchgehend etwa konstante Innenweite aufweist, wobei vorzugsweise der Pumpenzylinder (23) der Pumpe (20) an dem einen im Querschnitt bügelförmigen, insbesondere kappenförmigen Bauteil (4) befestigt und/oder mit Radialabstand vom Kappen-Mantel (12) auf dem Kappenboden stehend angeordnet ist.

6. Austragvorrichtung nach einem der vorhergehenden Ansprüche, dadurch gekennzeichnet, daß zur Halterung eines Pumpenzylinders (23) der Pumpe (20) eine insbesondere am Außenumfang angreifende Aufnahme (35), vorzugsweise eine durch eine Innenhülse (31) gebildete Schiebeführung vorgesehen ist, die radial nach innen vor stehende Zentrier-Rippen (36) aufweist.

7. Austragvorrichtung nach einem der vorhergehenden Ansprüche, dadurch gekennzeichnet, daß die Arretierung (10) für denselben Nocken (42) mindestens zwei seitlich gegeneinander versetzte Abschnitte einer Längsführung (38, 39) aufweist, von denen mindestens eine mit ihrem Ende (41) einen Teilhub begrenzt, wobei benachbarte Längsführungen über eine Querführung (40) für den Nocken (42) verbunden sind, der insbesondere eine Arretierung (46) in mindestens einer Pumphub-Stellung, wie der Ausgangsstellung, bildet.

8. Austragvorrichtung nach einem der vorhergehenden Ansprüche, dadurch gekennzeichnet, daß die beiden Bauteile (3, 4) am Ende mindestens eines Teilhubes im wesentlichen gegeneinander verdrehgesichert zur Ausgangsstellung, insbesondere mit mindestens einer Rückstellfeder (37), rückführbar und/oder in der Ausgangsstellung gegen eine weitere Rückstellbewegung durch unmittelbaren Eingriff ineinander gesichert sind, wobei vorzugsweise innerhalb eines Außengehäuses (5) zwei ineinandergreifende Innenhülsen (30, 31) oder dgl. der beiden Bauteile (3, 4) vorgesehen sind, die am Außenumfang eine Rückstellfeder (37) führen, Rückstell-Anschläge (32, 33) aufweisen und/oder ein Innengehäuse (29) für die Pumpe (20) bilden.

9. Austragvorrichtung nach einem der vorhergehenden Ansprüche, dadurch gekennzeichnet, daß die beiden Bauteile (3, 4) über eine Schnappverbindung (34) miteinander verbunden sind.

10. Austragvorrichtung nach einem der vorhergehenden Ansprüche, dadurch gekennzeichnet, daß einer der Bauteile (3, 4) mit einem offenen Pumpenzylinder (23) eine Montageeinheit bildet, die an dem anderen, vorzugsweise mit einem frei ausragenden Pumpkolben (21) eine Montageeinheit bildenden Bauteil (3) durch mindestens eine Steckverbindung gehaltert ist.

11. Austragvorrichtung nach einem der vorhergehenden Ansprüche, dadurch gekennzeichnet, daß die Außenbegrenzung der Austragvorrichtung (1) nur durch ein zweiteiliges Außengehäuse (5) und einen Austragstutzen (13) gebildet ist, der einteilig von einer Stirnwand (8) des Außengehäuses (5) vorsteht und vorzugsweise wesentlich kleinere Weite als das Außengehäuse (5) hat.

## Claims

1. Discharge apparatus for media with a manually operable pump (20) operable for each overall pump stroke between a starting position and a pump stroke end position in partial strokes of two components (3, 4) of a casing (5) movable against one another and at the end of at least one partial stroke on arresting means (10) fixing the pump stroke is provided and which can be released directly following the partial stroke and which is formed from cooperating projections (42) and recesses (39) of the components (3, 4) of the casing (5), the projections and recesses being located in concealed manner in the interior of the space bounded by the casing (5).

2. Discharge apparatus according to claim 1, characterized in that the arresting means (10) is provided for two pressure handles (43, 44) for pump operation, whose pressure faces are at right angles to a pump axis (45) and/or are located on directly interengaging components (3, 4), that preferably the arresting means (10) is provided between two cap-like casing parts (6, 7), which in particular interengage with oppositely directed cap jackets (11, 12) and/or form handles (43, 44) with the cap end walls (8, 9) and that preferably the arresting means (10) is associated with a casing part (7) of an outer casing (5), which forms a rear end part of the discharge apparatus (1), engages telescopically in a front casing part (6) and/or forms a rear end face extending substantially over the entire cross-section of the discharge apparatus.

3. Discharge apparatus according to claim 1 or 2, characterized in that the arresting means (10) is provided in the vicinity or a rear end of the discharge apparatus (1) and/or behind a front end of a pump chamber (25), to which the arresting means (10) is in particular radially adjacent.

4. Discharge apparatus according to one of the preceding claims, characterized in that preferably the component (4) forming a rear end of the discharge apparatus (1) in the starting position projects over the component (3) arrestable with respect thereto, in the pump stroke end position, at least at the end of a partial stroke, is located substantially completely within said component (3) and in said position the components (3, 4) form in particular approximately aligned end faces.

5. Discharge apparatus according to one of the preceding claims, characterized in that the pump (20) is constructed as a thrust piston pump, with a pump piston (21) dispacebly engaging in a pump cylinder (23) and/or the pump cylinder (23) is a storage container (24), which is closed at the bottom (27), for the entire medium storage content for the pump (20), which is solely closed with the pump piston (21) and has a roughly constant inside width throughout and preferably the pump cylinder (23) of the pump (20) is fixed to one cross-sectionally, bow-shaped and in particular cap-like component (4) and/or is vertically positioned with the radial spacing from the cap jacket (12) on the cap bottom.

6. Discharge apparatus according to one of the preceding claims, characterized in that for the mounting support of a pump cylinder (23) of the pump (20) there is in particular a receptacle (35) engaging on the outer circumference, preferably a sliding guide formed by an inner sleeve (31), which has radially inwardly projecting centring ribs (36).

7. Discharge apparatus according to one of the preceding claims, characterized in that the arresting means (10) for the same cam (42) has at least two laterally, reciprocally displaced portions of a longitudinal guide (38, 39), whereof at least one bounds with its end (41) a partial stroke and adjacent longitudinal guides are connected by means of a transverse guide (40) for the cam (42) and which in particular forms an arresting means (46) in at least one pump stroke position, such as the starting position.

8. Discharge apparatus according to one of the preceding claims, characterized in that the two components (3, 4) at the ends of at least one partial stroke can be returned in substantially reciprocally turn-prevented manner to the starting position, particularly with at least one return spring (37) and/or are secured in one another in the starting position against a further return movement by direct engagement and preferably within an outer casing (5) are provided two interengaging inner sleeves (30, 31) or the like of the two components (3, 4), which in particular guide on the outer circumference a return spring (37), have return stops (32, 33) and/or form an inner casing (29) for the pump (20).

9. Discharge apparatus according to one of the preceding claims, characterized in that the two components (3, 4) are interconnected by means of a snap connection (34).

10. Discharge apparatus according to one of the preceding claims, characterized in that one of the components (3, 4) forms with an open pump cylinder (23) an assembly unit, which is mounted on the other component (3) preferably forming an assembly unit with a freely projecting pump piston (21), by at least one plug connection.

11. Discharge apparatus according to one of the preceding claims, characterized in that the outer boundary of the discharge apparatus (1) is only formed by a two-part outer casing (5) and a discharge connection (13), which projects in one piece from an end wall (8) of the outer casing (5) and preferably has a smaller width than the outer casing (5).

## Revendications

1. Distributeur pour matières comprenant une pompe manuelle (20), qui, par course de pompage totale comprise entre une position initiale et une position de fin de course, peut être actionné en faisant effectuer à deux éléments (3, 4) se mouvant l'un vers l'autre d'un carter (5) des courses partielles, et dans le cas duquel un dispositif d'arrêt (10) limitant la course de pompage est prévu à la fin d'au moins une partie de la course, lequel est placé de façon amovible directement à la suite de la course partielle et est constitué par des saillies (42) et des évidements (39) des éléments (3, 4) du carter (5) qui coopèrent entre eux, les saillies et les évidements étant dissimulés à l'intérieur de l'espace délimité par le carter (5).

2. Distributeur selon la revendication 1, caractérisé en ce que le dispositif d'arrêt (10) est prévu pour deux poussoirs (43, 44) destinés à actionner la pompe, dont les surfaces de pression sont placées transversalement par rapport à un axe de pompe (45) et/ou sur des éléments (3, 4) se mettant en prise directement l'un avec l'autre, en ce que le dispositif d'arrêt (10) est prévu, de préférence, entre deux parties de carter en forme de cuvette (6, 7), qui se mettent en prise l'une avec l'autre notamment au moyen de surfaces latérales de cuvette (11, 12) orientées en sens inverse l'une par rapport à l'autre et/ou forment des poussoirs (43, 44) avec des parois frontales de cuvette (8, 9), et en ce que le dispositif d'arrêt (10) est associé à une partie de carter (7) d'un carter externe (5), laquelle forme la partie d'extrémité arrière du distributeur (1), se loge de manière télescopique dans une partie de carter avant (6) et/ou forme une surface d'extrémité artière s'étendant pour l'essentiel sur l'ensemble de la section du distributeur.

3. Distributeur selon la revendication 1 ou 2, caractérisé en ce que le dispositif d'arrêt (10) est prévu dans la zone d'une extrémité arrière du distributeur (1) et/ou derrière une extrémité avant d'une chambre de pompage (25), le dispositif d'arrêt (10) étant disposé essentiellement à côté de cette dernière dans la direction radiale.

4. Distributeur selon l'une des revendications précédentes, caractérisé en ce que, de préférence, l'élément (4) formant en position initiale une extrémité arrière du distributeur (1) fait saillie de l'élément (3) pouvant être bloqué par rapport à lui et est situé, en position de fin de course, au moins à la fin d'une partie de course, essentiellement complètement à l'intérieur de cet élément (3), sachant que les éléments (3, 4) forment dans cette position notamment des surfaces d'extrémité approximativement en alignement l'une avec l'autre.

5. Distributeur selon l'une des revendications précédentes, caractérisé en ce que la pompe (20) est réalisée en tant que pompe alternative comportant un piston de pompage (21) en prise avec un cylindre de pompage (23) de manière à pouvoir s'y déplacer et/ou en ce que le cylindre de pompage (23) est un réservoir (24) fermé au niveau du fond (27) et destiné à toute la réserve de matière de la pompe (20), qui est seulement fermé par le piston de pompage (21) et qui présente une largeur intérieure approximativement constante sur toute son étendue, sachant, de préférence, que le cylindre (23) de la pompe (20) est fixé à l'un des éléments (4) notamment en forme de cuvette et présentant une section en forme d'étrier et/ou est posé sur le fond de la cuvette avec un écart radial par rapport à la surface latérale (12) de la cuvette.

6. Distributeur selon l'une des revendications précédentes, caractérisé en ce que, pour fixer un cylindre (23) de la pompe (20), est prévu(e) un logement (35) adjacent au pourtour extérieur, de préférence une glissière formée par une douille interne (31), laquelle présente des nervures de centrage (36) faisant saillie radialement vers l'intérieur.

7. Distributeur selon l'une des revendications précédentes, caractérisé en ce que le dispositif d'arrêt (10) présente pour la même saillie (42) au moins deux parties d'une glissière longitudinale (38, 39), qui sont décalées latéralement l'une par rapport à l'autre et dont au moins l'une délimite avec son extrémité (41) une course partielle, des glissières longitudinales adjacentes étant reliées par une glissière transversale (40) pour la saillie (42), cette dernière constituant notamment un dispositif d'arrêt (46) dans au moins une position de la course, telle que la position initiale.

8. Distributeur selon l'une des revendications précédentes, caractérisé en ce que les deux éléments (3, 4) sont essentiellement assurés l'un par rapport à l'autre contre la rotation à la fin d'au moins une course partielle, peuvent être ramenés dans la position initiale notamment par au moins un ressort de rappel (37) et/ou sont assurés, dans la position initiale, contre un mouvement de rappel supplémentaire en étant directement en prise l'un avec l'autre, sachant, de préférence, qu'à l'intérieur d'un carter externe (5) sont prévues deux douilles internes (30, 31) ou analogues des deux éléments (3, 4), lesquelles sont en prise l'une avec l'autre et guident sur le pourtour extérieur un ressort de rappel (37), présentent des butées de rappel (32, 33) et/ou forment un carter interne (29) pour la pompe (20).

9. Distributeur selon l'une des revendications précédentes, caractérisé en ce que les deux éléments (3, 4) sont reliés entre eux par une liaison par enclenchement (34).

10. Distributeur selon l'une des revendications précédentes, caractérisé en ce que l'un des éléments (3, 4) forme avec un cylindre ouvert (23) une unité de montage, qui est maintenue par au moins une liaison par emboîtement à l'autre élément (3) formant, de préférence avec un piston de pompage (21) faisant saillie librement, une unité de montage.

11. Distributeur selon l'une des revendications précédentes, caractérisé en ce que la limite extérieure du distributeur (1) n'est formée que par un carter externe en deux parties (5) et par une tubulure d'écoulement (13) qui fait saillie d'un seul tenant d'une paroi frontale (8) du carter externe (5) et dont la largeur est, de préférence, essentiellement inférieure à celle du carter externe (5).
